# EUROPEAN PATENT SPECIFICATION

(11) **EP 1 820 522 B1**
(45) Date of publication and mention of the grant of the patent: **16.05.2012**
(21) Application number: 06003422.0
(22) Date of filing: 20.02.2006
(51) Int. Cl.: A61L 31/12, A61L 31/14, A61L 27/12, A61L 27/50

(54) **Granulate-matrix**
Granulatematrix
Matrice à base de granulats

(43) Date of publication of application: 22.08.2007
(60) Divisional application: 10013455.0
(73) Proprietor: Straumann Holding AG, 4002 Basel (CH); Universität Zürich, 8006 Zürich (CH)
(72) Inventor: Fehr, Daniel, 8008 Zürich (CH); Neidhardt, Astrid, 8001 Zürich (CH); Molenberg, Aaldert, 4102 Binningen (CH); Jung, Roland, 8044 Zürich (CH); Hämmerle, Christoph, 8067 Zürich (CH)
(74) Representative: Schaad, Balass, Menzl & Partner AG

(56) References cited:
- EP-A- 0 324 425
- EP-A1- 1 609 491
- WO-A-00/44808
- WO-A-03/040235
- WO-A-03/092760
- WO-A-2004/054633
- WO-A-2004/103421
- US-A1- 2004 019 132
- US-A1- 2004 096 507

## Description

The present invention relates to a composition comprising a granulate and a matrix obtainable by a self selective reaction of at least two precursors forming a three dimensional network. A kit and a method for preparing said composition are also provided.

Medical devices such as implants in general and dental implants in particular are widely used nowadays. They have become an appreciated possibility where hard tissue structures need to be fixed or replaced, e.g. in the case of bone fractures or tooth loss. However, the success of such implants strongly depends on adequate support at the implant site. If the bone mass at said site is insufficient or poor in quality, bone repair and/or bone augmentation becomes a necessity. There are different treatments applied to regain sufficient bone mass, including the use of bone graft materials of different origin, shape and size.

While there are ways to systemically treat the mass and/or strength of the bone, e.g. in osteoporosis, it is still difficult to achieve bone formation in a reliable and controllable manner. However, local bone formation would greatly benefit the adequate treatment of incidents where enhancing the bone volume is only required locally, e.g. when placing dental implants.

Methods currently used to repair bone defects include graft materials from different sources. The material is either synthetic or of natural origin. One natural graft material which is employed is autogenous bone. In contrast to bone/bone like material from natural sources (human, animals, plants, algae etc.), autogenous bone material does not trigger a strong immune response and is thus not rejected by the host. However, autogenous bone material requires a second surgery for harvesting the bone increasing the risk of unwanted infection and/or inflammation at this site and significantly increases treatment costs. Further, the removal of bone material leads, at least temporarily, to a weakened structure at this site and causes a painful healing process.

During the last years it became more and more clear that the use of various bioactive factors improves bone repair and/or bone augmentation. It has also been shown that the method of application of such factors greatly influences their regenerative effect. Despite continuous efforts to develop methods for the controllable presentation and release of said factors, this is still one of the common problems in this field.

In the state of the art, different biomaterials for tissue augmentation or release of bioactive factors have been described.

WO 00/44808 discloses a polymeric biomaterial formed by nucleophilic addition reactions to conjugated unsaturated groups. The obtained biomaterial, which is in the form of a hydrogel, may be used for example as glues or sealants and as scaffolds for tissue engineering and wound healing applications. Also said hydrogels degrade fast under physiological conditions.

US 5,626,861 discloses a method for the fabrication of a macroporous matrix that may be used as implant material. The composites are formed from a mixture of biodegradable and biocompatible polymer which is dissolved in an organic solvent such as methylene chloride or chloroform and then mixed with hydroxyapatite. The latter is a particulate calcium phosphate ceramic. The material has irregular pores in the size range between 100 and 250 microns. Bioactive factors may be non-covalently incorporated in the composite.

US 5,204,382 describes injectable implant compositions comprising a biocompatible ceramic matrix mixed with an organic polymer or collagen suspended in a fluid carrier. The ceramic particles are in the size range of 50µm to 250µm.

US 6,417,247 discloses polymer and a ceramic matrix. The compositions are normally liquid and harden upon a certain stimulus, e.g. elevated temperatures.

As used herein, the words "polymerization" and "cross-linking" are used to indicate the linking of different precursors to each other to result in a substantial increase in molecular weight. "Cross-linking" further indicates branching, typically to obtain a three dimensional polymer network.

By "self selective" is meant that a first precursor A of the reaction reacts much faster with a second precursor B than with other compounds present in the mixture at the site of the reaction, and the second precursor B reacts much faster with the first precursor A than with other compounds present in the mixture at the site of the reaction. The mixture may contain other biological materials, for example, drugs, peptides, proteins, DNA, RNA, cells, cell aggregates and tissues.

By "conjugated unsaturated bond" the alternation of carbon-carbon, carbon-heteroatom or heteroatom-heteroatom multiple bonds with single bonds is meant. Such bonds can undergo addition reactions.

By "conjugated unsaturated group" a molecule or a region of a molecule, containing an alternation of carbon-carbon, carbon-heteroatom or heteroatom-heteroatom multiple bonds with single bonds, which has a multiple bond which can undergo addition reactions is meant. Examples of conjugated unsaturated groups include, but are not limited to acrylates, acrylamides, quinines, and vinylpyridiniums, for example 2- or 4-vinylpyridinium.

The problem of the present invention is to provide a bone repair and/or bone augmentation material which has an excellent biocompatibility and mechanical stability allowing in situ repair of the bone defect and/or bone augmentation while minimizing the risk of unwanted inflammation, eliminating the need for second surgery for harvesting autogenous bone material and not bearing the risk of infection. In addition, the treatment costs are significantly reduced.

The problem is solved by a composition according to claim 1. Further preferred embodiments are subject of claims 2 to 28.

The composition according to the present invention comprises a granulate and a degradable polymeric matrix as disclosed in claim 1. Several cross-linked substances are known in the art, which are able to provide a porous three-dimensional biodegradable matrix suitable for tissue regeneration obtainable by a self selective reaction. An example for a polymeric material is PEG.

In the preferred embodiment, such polymeric matrix is obtained by a self selective reaction of two or more precursors, as defined below, in the presence of water. The combination of said granulate and said matrix yields a composition having excellent bone repair and/or bone augmentation properties. The combination of said matrix with said granulate synergistically improves the bone repair and/or bone augmentation. While the matrix provides a three-dimensional scaffold, the granulate ensures a good mechanical stability. Since precursors forming the matrix and granulate are mixed just prior to use, an optimal distribution of the granulate throughout the entire composition is achieved. The precursors, which are the monomers forming the matrix, are soluble in water. It is important to note that precursors and not polymers are mixed with the granulate allowing the formation of the matrix in situ. Consequently, the aqueous solution comprising the precursors and the granulate is not viscous and can be rapidly mixed without difficulties. The rapid generation of the matrix preserves the optimal distribution of the granulate and avoids imbalances due to possible sedimentation of the granulate.

Furthermore, the combination of a hydrogel matrix and granulate allows modelling of the granular putty to the desired shape, stabilizes the shape and prevents granulate migration.

If appropriate, a viscosity modifier, such as CMC (carboxymethylcellulose), PGA (propylene glycol alginate) or Xanthan, can be added to ensure optimal physical properties for administration in situ, e.g. in case a relatively large amount of liquid should be added to the granules. Thus, uniform and optimal bone repair and/or bone augmentation properties are ensured throughout the entire three-dimensional structure formed by the composition.

In previously known treatments, the bone filler material is applied upon mixing with non polymerizing liquids, e.g. NaCl solutions or blood. As a result, the administered bone grafting mixture may not provide for an accurate stability required for successful new hard tissue formation. The bone graft material is usually exposed to mechanical stress due to the overlying layer of soft tissue or other impacts, which can lead to the deformation, migration or even collapse of the augmentate.

The composition of the present invention will overcome this problem by the combination of an appropriate filler material, e.g. calcium phosphate granulate, and a polymeric matrix, e.g. PEG, and thereby provide for controlled and safe bone repair and/or bone augmentation.

Apart from the simple handling, the single components of the composition, the precursors forming the matrix and the granulate, have an excellent stability and thus a long shelf life. Advantageously, the components are stored in a dry form, e.g. as a powder, and the precursors are dissolved immediately prior to application. Alternatively, the components may be stored in solvents that protect their functionalities.

Further, the composition is biodegradable thereby leaving space for natural bone to grow into. Again, this avoids surgery in order to remove remaining parts of the bone repair and/or bone augmentation material subsequently to the completed healing of the bone defect. The degradation products are easily excreted and non-toxic.

The granulate serves on one hand as a filler expanding the volume of the composition and, on the other hand, it provides the necessary mechanical strength of the composition. Furthermore, it offers a scaffold surface for bone deposition. There is a wide variety of materials which can be employed as granulate, e.g. bone materials or synthetic materials. Examples of granulate materials are autograft bone, hydroxyapatite, tricalcium phosphate and mixtures thereof.

Further examples of granulate materials for autogenous bone materials are chin, retromolar and nasal spine (all harvested intraorally), crista, iliaca and calotte (all harvested extraorally), bone/bone like materials from natural sources are freeze dried bone allograft (FDBA), demineralized freeze dried bone allograft (DFDBA; Grafton®), bovine material (BioOss®, Osteograph®, Navigraft®, Osteograft®), coralline material (Pro Osteon®, Interpore 500®), algae material (Frios Algipore®), collagens. Synthetic materials are hydroxyapatite (Ostim®), tricalciumphosphate (Cerasorb®, BioResorb®, Ceros® etc.), mixtures of hydroxyapatite and tricalciumphosphate (Straumann BoneCeramic®), bioactive glass (PerioGlas®, Biogran®), calcium sulfate and carbonated apatite.

The synthetic materials provide the advantage that they are of non-animal origin, thus eliminating the risk of infection with human or animal pathogens, depending on the source of the natural materials, which is always present when not autogenous bone material but bone/bone like materials from natural sources are used. In addition, synthetic granulates eliminate the need for a second surgery, in contrast to the case when autogenous bone material is employed. Said second surgery is a prominent source of complications and additional costs. Apart from the fact that sound bone structures are at least temporarily weakened, infections or inflammation may occur, further complicating the healing process of the surgery site which itself is already painful.

Another advantage of synthetic materials is, that its manufacturing allows to control parameters such as chemical composition, crystallinity, porosity.

Below, precursors A and B forming the matrix are described in more detail.

The first precursor A comprises a core which carries n chains with a conjugated unsaturated group or a conjugated unsaturated bond attached, said conjugated unsaturated group or conjugated unsaturated bond is terminal. The core of the first precursor A can be a single atom such as a carbon or a nitrogen atom or a small molecule such as an ethylene oxide unit, an amino acid or a peptide, a sugar, a multifunctional alcohol, such as pentaerythritol, D-sorbitol, glycerol or oligoglycerol, such as hexaglycerol. The chains are linear polymers or linear or branched alkyl chains optionally comprising heteroatoms, amide groups or ester groups. Beside the chains, the core of precursor A may be additionally substituted with linear or branched alkyl residues or polymers which have no conjugated unsaturated groups or bonds. In a preferred embodiment the first precursor A has 2 to 10 chains, preferably 2-8, more preferably 3-8, most preferably 4-8 chains. The conjugated unsaturated bonds are preferably acrylates, acrylamides, quinines, 2- or 4-vinylpyridiniums, vinylsulfone, maleimid or itaconate esters of formula Ia or Ib 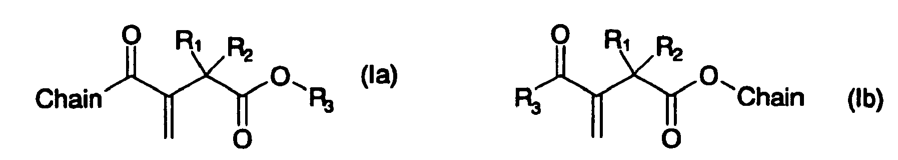 wherein R₁ and R₂ are independently hydrogen, methyl, ethyl, propyl or butyl, and R₃ is a linear or branched C₁ to C₁₀ hydrocarbon chain, preferably methyl, ethyl, propyl or butyl.

The second precursor B comprises a core carrying m chains each having a thiol group which is terminal. For example a cysteine residue may be incorporated into the chain. The core of the second precursor B can be a single atom such as a carbon or a nitrogen atom or a small molecule such as an ethylene oxide unit, an amino acid or a peptide, a sugar, a multifunctional alcohol, such as pentaerythritol, D-sorbitol, glycerol or oligoglycerol, such as hexaglycerol. The chains are linear polymers or linear or branched alkyl chains optionally comprising heteroatoms, esters groups or amide groups. In a preferred embodiment the second precursor B has 2 to 10 chains, preferably 2-8, more preferably 2-6, most preferably 2 to 4 chains.

In a preferred embodiment, the core of precursor B comprises a peptide which comprises one or more enzymatic degradation sites. Examples for enzymatic degradation sites are substrate sequences for plasmin, matrix metallo-proteinases and the like.

In a preferred embodiment, precursor A and/or B comprises a peptide which comprises one or more enzymatic degradation sites. Precursor A and/or B can also be a peptide comprising 2 cystein residues and one or more enzymatic degradation sites. Such precursors are described in WO 03/040235. Examples for enzymatic degradation sites are substrate sequences for plasmin, matrix metallo-proteinases and the like.

In a preferred embodiment a precursor which comprises a peptide or is a peptide comprising 2 cystein residues and one or more enzymatic degradation sites as described for precursor B can be used as a third precursor.

The first precursor A compound has n chains, whereby n is greater than or equal to 2, and the second precursor B compound has m chains, whereby m is greater than or equal to 2. The first precursor A and/or the second precursor B may comprise further chains which are not functionalized. The sum of the functionalized chains of the first and the second precursor, that means m+n, is greater than or equal to 5. Preferably the sum of m+n is equal to or greater than 6 to obtain a well formed three-dimensional network.

The precursors forming the matrix are dissolved or suspended in aqueous solutions. The precursors do not necessarily have to be entirely water-soluble.

The granulate can be wetted with the precursor solutions or suspended in a larger amount of precursor solutions.

Since no organic solvents are necessary, only aqueous solutions and/or suspensions are present. These are easy to handle and do not require any laborious precautions as might be the case if organic solvents are present. Further, organic solvents are an additional risk for the health of the staff and the patients exposed to these solvents. The present invention eliminates said risk.

The use of at least two precursors which form a three dimensional network by a self selective reaction can advantageously be applied in situ. This means, the composition is brought to the site of the bone defect in form of a liquid or paste, allowing a precise control of the amount of composition applied. The still liquid composition optimally adopts the shape of the bone defect, ensuring optimal fit and hold. Furthermore, it allows modelling of the composition to the desired shape. No further fixation is needed. The hardening of the composition is completed within minutes, starting at the time of mixing. It does not require any complicated triggering stimulus and the self selectivity of the reaction is such that surrounding tissue is not harmed.

In a preferred embodiment the granulate comprises calciumphosphate, which is highly biocompatible in terms that it is inert, i.e., does not elicite inflammatory processes or further unwanted biological reactions.

In a further preferred embodiment the granulate comprises hydroxyapatite (HA) and/or tricalciumphosphate (TCP).

In a preferred embodiment the composition comprises a granulate wherein the weight ratio of hydroxyapatite/tricalciumphosphate in the granulate is between 0.1 to 5.0, preferably between 1.0 to 4.0, most preferably between 1.0 to 2.0.

In another preferred embodiment the content of hydroxyapatite (HA) in the granulate is at least 1% by weight, preferably equal to or more than 15% by weight, most preferably equal to or more than 50% by weight.

The mechanical strength of the composition is greatly influenced by the amount of granulate present in the composition. Good results are achieved with compositions comprising 30% to 60% by weight granulate.

In a further preferred embodiment the conjugated unsaturated group or the conjugated unsaturated bond of first precursor A is an acrylate, a quinine, a 2- or 4-vinylpyridinium, vinylsulfone, maleimide or an itaconate ester of formula Ia or Ib.

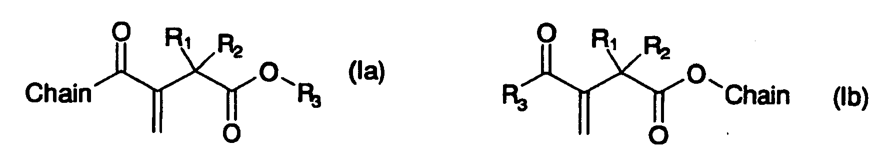

Most preferred are acrylates.

In a particularly preferred embodiment precursor A is chosen from the group consisting of

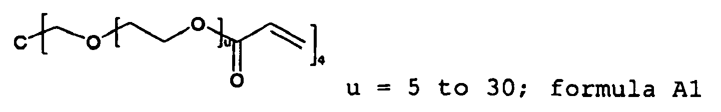

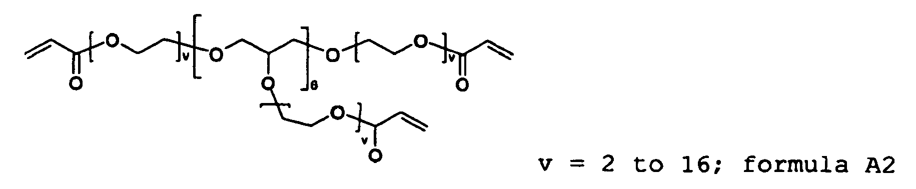

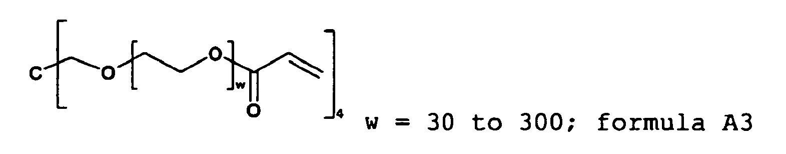

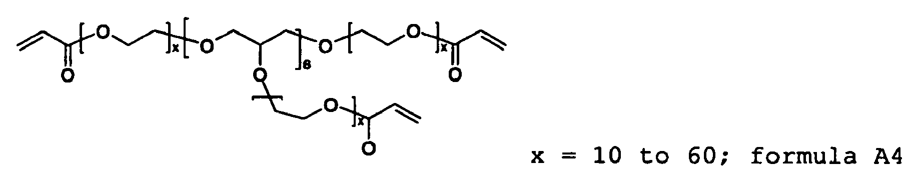

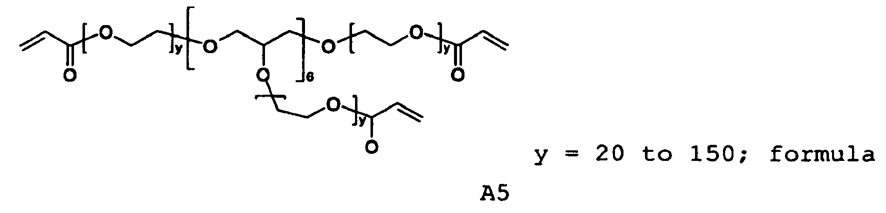

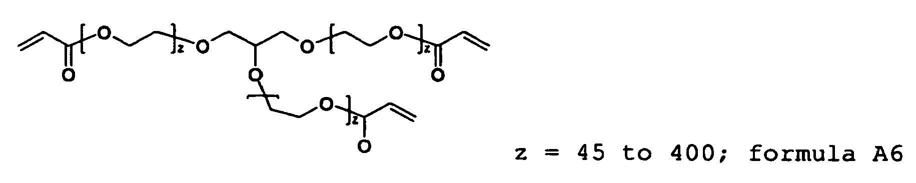

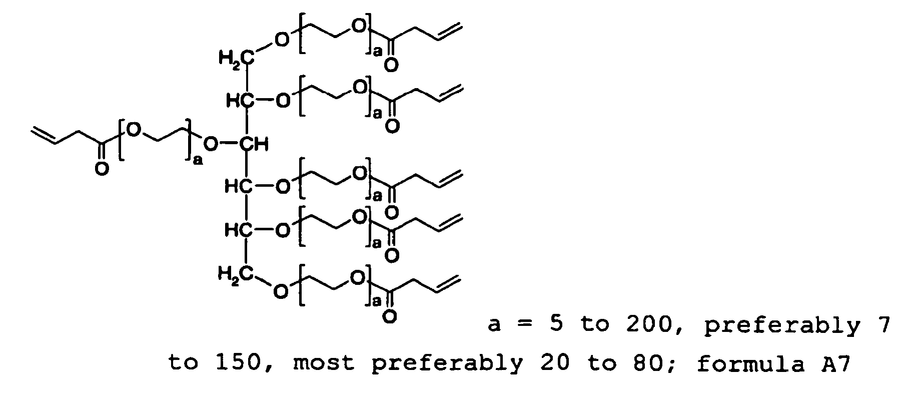

In another preferred embodiment precursor B comprises a thiol moiety or is selected from the group consisting of

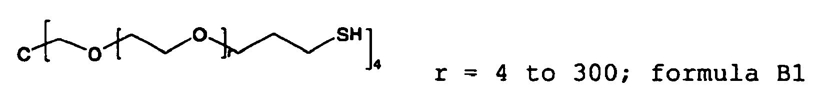

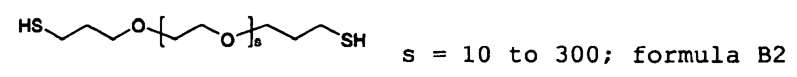

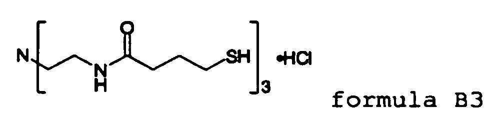

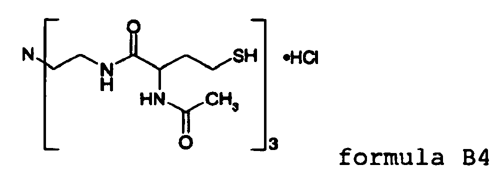

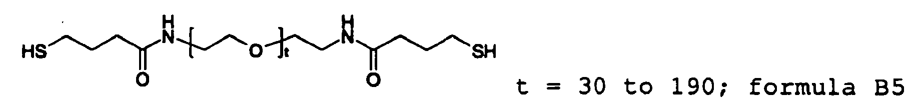

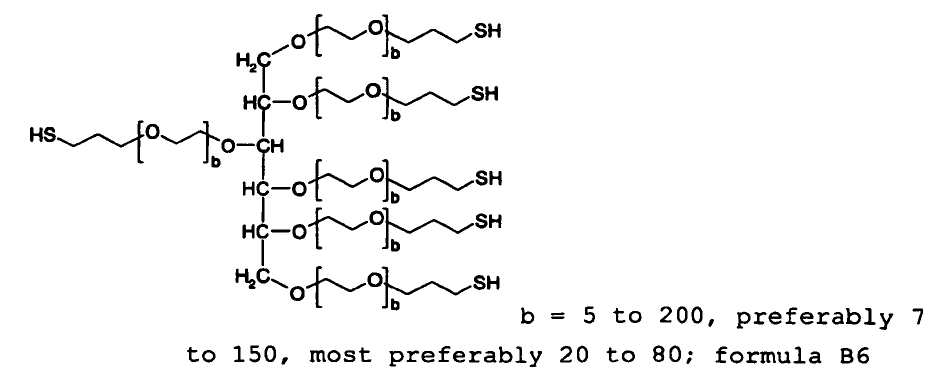

Most preferred precursor A is a PEG-acrylate carrying 4 chains and having a molecular weight of approximately 15'000 Da. Most preferred precursors B are selected from the group consisting of a linear PEG-dithiol having a molecular weight of approximately 3500 Da and PEG-thiol carrying 4 chains and having a molecular weight of about 2400 Da.

Precursor A and/or B can significantly vary in their molecular weight, preferably in the range of 500 Da to 100'000 Da, more preferably in the range of 1000 to 50'000 and most preferably in the range of 2000 to 30'000.

In a preferred embodiment the chains of precursor A and/or B are a polymer selected from the group consisting of poly(vinyl alcohol), poly(alkylene oxides), poly(ethylene glycol), poly(oxyethylated polyols), poly(oxyethylated sorbitol, poly(oxyethylated glucose), poly(oxazoline), poly(acryloyl-morpholine), poly(vinylpyrrolidone), and mixtures thereof. In a particularly preferred embodiment the chains of precursor A and/or B are poly(ethylene glycol). The poly(ethylene glycol) can be either linear or branched.

In another preferred embodiment precursor A is used with a precursor B which is a peptide comprising 2 cystein residues and one or more enzymatic degradation sites. The cystein residues are preferably located at the termini of the peptide.

In a preferred embodiment the composition comprises at least one bioactive factor. The bioactive factor can be added when mixing the other components of the composition. If the bioactive factor does not comprise a reactive group, e.g. a thiol or an amine group, said bioactive factor will not be covalently bound to the matrix, but simply be entrapped in the composition. The bioactive factor is then released by diffusion. However, the factor may also be covalently bound to the matrix, e.g., this can be achieved by a thiol moiety present in the bioactive factor which reacts with the conjugated unsaturated group or bond present in precursor A upon mixing. A thiol moiety is present, e.g. in the amino acid cystein. This amino acid can easily be introduced in peptides, oligo-peptides or proteins. It is also possible to adsorb the bioactive factor on the granules prior to the mixing of the granules with solutions comprising the first precursor A and the second precursor B.

In a preferred embodiment the bioactive factor is selected from the group consisting of parathyroid hormones (PTH), peptides based on PTH, peptide fragments of PTH, peptides comprising an RGD tripeptide, transforming growth factor beta family (TGFβ), bone morphogenetic protein family (BMP), platelet derived growth factor family (PDGF), vascular endothelial growth factor family (VEGF), insulin like growth factor family (IGF), fibroblast growth factor family (FGF), enamel matrix derivative proteins and peptides (EMD) as described in EP 01165102 B1, prostaglandin E₂ (PGE₂) and EP2 agonists, and dentonin. Dentonin is a peptide fragment of matrix extracellular phosphoglycoprotein (MEPE) found in bone and dental tissues. It is further described in WO 02/14360. Also, extracellular matrix proteins, such as fibronectin, collagen, laminin, may be used as bioactive factors. These peptides and proteins may or may not comprise additional cystein. Such cystein facilitates the covalent attachment of the peptides and proteins to the matrix.

In another preferred embodiment the bioactive factor is selected from the group consisting of parathyroid hormones (PTH), peptides based on PTH and peptide fragments of PTH. Parathyroid hormones have been shown to exert multiple anabolic effects on bone tissue. Particularly preferred is a peptide comprising the first 34 amino acids of PTH. This peptide may or may not contain an additional cystein, which facilitates the covalent attachment of the peptide to the matrix. Such peptides can be produced by enzymatic cleavage of PTH or by peptide synthesis. In a further preferred embodiment the bioactive factor is selected from the group consisting of amelogenin, amelin, tuftelin, ameloblastin, enamelin and dentin sialoprotein.

The effectiveness of the matrix can be enhanced by introduction of cell attachment sites. For exemple, the RGD sequence motif plays an important role in specific cell adhesion. A possible cell attachment peptide is H-Gly-Cys-Gly-*Arg*-*Gly*-*Asp*-Ser-Pro-Gly-NH₂, which can be covalently attached to the matrix through its cystein.

The bioactive factors may be prepared from natural sources, by synthetic or recombinant means or a mixture thereof.

According to claim 29 the present invention also relates to kits for preparing a composition according to claim 1 to 28. Preferred embodiments are the subject-matter of claims 29 to 35.

Kits also fall within the scope of the present invention. Such a kit is used to prepare a composition according to the present invention. The kit comprises (i) a granulate, (ii) a precursor A and (iii) a precursor B which are each individually stored. The kit may also comprise more than one granulate and more than two precursors.

In a preferred embodiment the kit also comprises at least one bioactive factor as a further component (iv) which is individually stored as well. If desired, the kit may comprise two or more bioactive factors stored as premix or, preferably, individually stored. In the latter case, the factors can be mixed when the kit is used according to specific needs of the patient.

It is also possible that the kit comprises certain components in premixed form. For instance, the granulate and precursor A can be stored as premix, the granulate and precursor B can be stored as premix and also precursor B and the bioactive factor can be stored as premix. The precursors can be stored in dry form or in a suitable solvent (e.g. 0.04% acetic acid). A suitable buffer solution is added immediately prior to application. The precursors are preferably stored in a dry form. The bioactive factor can be (pre-)adsorb to the granulate. Further, the bioactive factor can be stored in a dry (lyophilized) form or in an aqueous solution which is suitably buffered. The former provides excellent stability and thus a long shelf life, the latter provides a very user-friendly handling.

According to claim 36 and 37 the present invention also relates to a method for preparing a composition according to claims 1 to 26.

A method for preparing a composition according to the present invention is also provided. For this purpose, the granulate, the precursor A and the precursor B are mixed in the presence of water. Preferably, the water is buffered near or at the physiological pH. A suitable buffering range for the matrix is pH 7.4 to 9.0. The polymerization starts upon mixing of the different components and a hydrogel is formed within a quite short period of time (10 seconds up to 10 minutes). The precursors do not necessarily have to be completely water soluble.

The mixing of the different components can be achieved in several ways. If the precursors A and B are stored as aqueous solutions they can be mixed with the granulate by means of a suitable mixing device. Preferably they are filter sterilized just prior to their use. Most preferably, the components are sterilized at the time of production and packed in such a way that sterility is preserved. If the components are stored in powder form, they can each be dissolved in an appropriate buffered aqueous solution.

If the kit comprises a bioactive factor, the factor may be premixed or pre-reacted with any of the precursors or added separately in dry or lyophilized form or dissolved state. For instance, if the bioactive factor comprises a thiol, it can be pre-reacted with precursor A. The bioactive factor can also be preadsorbed to the granulate prior to mixing with the precursors A and B.

According to claim 38 the present invention also relates to the use of a composition according to claim 1 to 28 as material for bone repair and/or bone augmentation.

In a preferred embodiment the composition according to the present invention is used as bone repair and/or bone augmentation material.

### Example 1

164 mg (0.084 mmol thiol) of HS-PEG-SH 3.4 k (Nektar, Huntsville, AL, USA) were dissolved in 1.71 ml of 0.05% acetic acid and 326 mg (0.083 mmol acrylate) of 4-arm PEG-acrylate 15k (Nektar, Huntsville, AL, USA) were dissolved in 1.55 ml of 0.05% acetic acid containing 100 ppm of methylene blue. Mixing aliquots of both PEG solutions with a 0.4 M triethanolamine/HCl buffer (pH 8.85) in a volume ratio of 1.5 : 1.5 : 1 yielded a gel in 3.5 minutes at 25°C.

Aliquots of the three solutions (V_{PEG-thiol} : V_{PEG-acrylate} : V_{buffer} = 1.5 : 1.5 : 1) were pipetted to HA/TCP (60%/40%) granules (Straumann Bone Ceramic, Institut Straumann AG, Basel, Switzerland) and mixed. Three surgeons independently evaluated the application properties of compositions with various granules / liquid ratios:

| Granules (g) | Liquid (ml) | Surgeon 1 | Surgeon 2 | Surgeon 3 |
|---|---|---|---|---|
| 0.5 | 0.6 | good consistency | slightly too little liquid | good application properties |
| 0.5 | 0.7 | best consistency, all liquid is absorbed | good application properties | good application properties |
| 0.5 | 0.9 | good consistency, some liquid not absorbed | - | - |

The tests showed that the granules readily absorbed the PEG solution and the resulting granular putty was easy to apply in a mandibular defect model and yielded a stable augmentate after gelation of the PEGs.

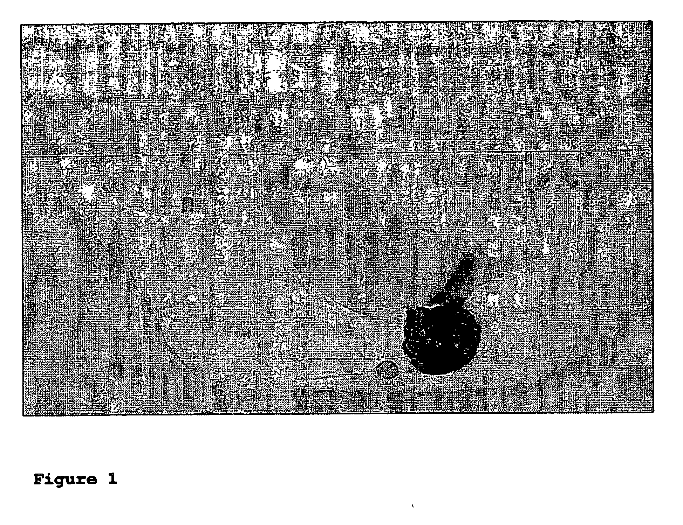

Figure 1 shows a mandibular defect model with granular putty after gelation, applied by surgeon 1

### Example 2

### Formulation 1:

150 mg (0.47 mmol acrylate) of 8-arm PEG-acrylate 2k were dissolved in 0.60 ml of 0.02 M triethanolamine/HCl buffer (pH 7.6) and 311 mg (0.49 mmol thiol) of 4-arm PEG-thiol 2k were dissolved in 0.44 ml of water.

Mixing equal aliquots of both solutions yielded a gel in ca. 35 seconds at 37°C.

### Formulation 2:

170 mg (0.45 mmol acrylate) of 6-arm PEG-acrylate 2k were dissolved in 0.58 ml of 0.05 M triethanolamine/HCl buffer (pH 9.8) and 190 mg (0.47 mmol thiol) of 6-arm PEG-thiol 2k were dissolved in 0.56 ml of water.

Mixing equal aliquots of both solutions yielded a gel in ca. 75 seconds at 37°C.

### Formulation 3:

69 mg (0.018 mmol acrylate) of 4-arm PEG-acrylate 15k were dissolved in 0.131 ml of 0.04% aqueous acetic acid containing 100 ppm methylene blue and 11 mg (0. 018 mmol thiol) of 4-arm PEG-thiol 2k were dissolved in 0.189 ml of 0.04% aqueous acetic acid.

Mixing aliquots of both PEG solutions with a 0.05 M triethanolamine/HCl buffer (pH 8.7) in a volume ratio of 1 : 1 : 3 yielded a gel in ca. 2.5 minutes at 25°C.

Mixing any of the above 3 formulations with HA/TCP (60%/40%) granules (Straumann Bone Ceramic, Institut Straumann AG, Basel, Switzerland) yields a granular putty with similar application properties as those of the formulation of example 1.

### Example 3

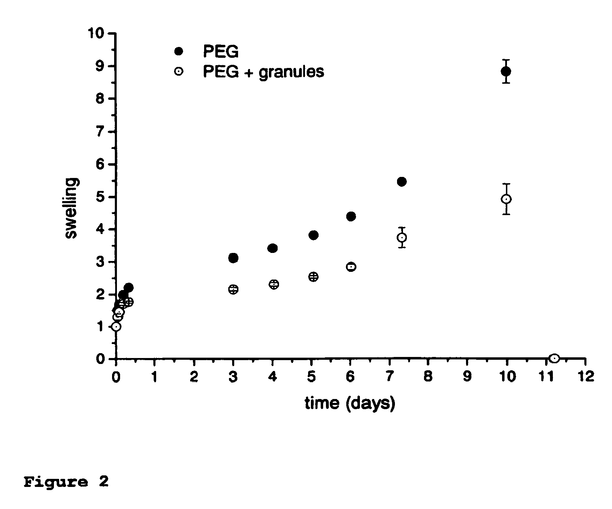

A 0.1 M aqueous solution of triethanolamine was brought to pH 8.7 using 2 M hydrochloric acid. 4-arm PEG-acrylate 15k and HS-PEG-SH 3.4 k (both from Nektar, Huntsville, AL, USA) were dissolved in this buffer solution, such that the total PEG concentration was 9.8 wt% and equimolar amounts of acrylate and thiol groups were present. Half of the solution was mixed with HA/TCP (60%/40%) granules in a ratio of 0.6 ml liquid per 0.5 g granules. From both the PEG solution and the mixture of PEG solution with granules, 6 cylindrical gels with a diameter of 6 mm were cast using stainless steel molds. After curing for 15 min, the gels were weighed, added to a Falcon tube containing 10 ml of 30 mM PBS (pH 7.4) and placed in a water bath at 37°C. At regular intervals the gels were taken from the buffer solution, blotted dry, and weighed. The pH of the buffer solution was checked and, if the value deviated by more than 0.1 from pH 7.4, the buffer was replaced by fresh 30 mM PBS (pH 7.4). The disintegration of the gels was followed by dividing their weight at each time point by the weight immediately after casting. Both the gels with and those without granules degraded at the same rate and had completely degraded within ca. 11 days (figure 2), however, the addition of granules led to a markedly lower swelling.

Figure 2 shows the swelling of the hydrogel samples (9.8 wt% PEG with/without granules) against time in PBS (pH 7.4) at 37°C. Average values of 6 samples (± SD) are given.

### Example 4

### Methods

16 adult (12 months old) New Zealand White rabbits, weighing between 3 and 4 kg, were anesthetized and obtained each 4 titanium cylinders of 7 mm in height and 7 mm in outer diameter, which were screwed in 1 mm deep circular perforated slits made in the cortical bones of the calvaria. The following 4 treatment modalities were randomly allocated: (1) empty control, (2) a combination of PEG matrix and hydroxyapatite (HA) / tricalciumphosphate (TCP) granules (Straumann Bone Ceramic; Institut Straumann AG, Basel, Switzerland), and a combination of PEG matrix containing either 100 (3) or 20µg/g gel (4) of PTH₁₋₃₄ and HA/TCP granules. Immediately before application, 4-arm PEG-acrylate 15k and HS-PEG-SH 3.4 k (both from Nektar, Huntsville, AL, USA) were each dissolved in a 0.1 M aqueous triethanolamine/HCl buffer (pH 8.7), such that the total PEG concentration in both solutions together was 9.8 wt% and equimolar amounts of acrylate and thiol groups were present. Both PEG solutions were then sterile filtered. For the activated gels, a 35 amino acid peptide of the parathyroid hormone (cys-PTH₁₋₃₄) and a 9 amino acid cys-RGD peptide (both from Bachem, Bubendorf, Switzerland) were additionally added to the PEG-acrylate solution, resulting in the formation of covalent bonds between the cystein-residues and the PEG-acrylate. The final concentrations for the peptides were 350 µg/g gel for cys-RGD and 20 or 100 µg/g gel for cys-PTH₁₋₃₄.

The PEG solutions were then applied onto the HA/TCP granules and mixed for about 10 seconds. Subsequently, this granular putty was applied into the determined cylinders. Within 60 seconds, the PEG gels set and thus stabilized the HA/TCP granules. The cylinders were left open towards the bone side but were closed with a titanium lid towards the covering skin-periosteal flap. The periosteum and the cutaneous flap were adapted and sutured for primary healing.

After 8 weeks, the animals were sacrificed and ground sections were prepared for histology.

The bone formation in the cylinders was evaluated histologically. Mean values and standard deviations were calculated for the amounts of bone formation within the cylinders, either evaluated by the point measurements or by the area of bone regeneration and for the graft to bone contact. For statistical analysis, repeated measures ANOVA and subsequent pairwise Student's t-test with corrected p-values according to Holm's were used to detect the differences between the 4 treatment modalities.

### Results

All animals showed uneventful healing of the area of surgery and no reductions in body weights were noted. Upon specimen retrieval, 3 cylinders were dislocated from the skull bone because of loss of fixation and were embedded in soft connected tissue. These 3 cylinders, 2 test sites and one control site, were excluded from further analysis. The remaining 61 cylinders were found to be stable and in the same position as at placement.

Qualitative histological evaluation revealed varying amounts of newly formed bone with no signs of inflammation in all cylinders. In the empty control cylinders, the augmented tissue comprised of slender bone trabeculae and large marrow spaces. The bone trabeculae adjacent to the surface of the inner wall of the cylinders were oriented parallel to and in various degrees of intimate contact with the surface of the machined cylinders.

The amount of newly formed bone within the control cylinders containing the unfunctionalized PEG matrix and the HA/TCP granules alone varied greatly. In contrast to the empty cylinders, the bone growth was not dominantly along the titanium walls and new bone was mostly in intimate contract with the granulate, which appeared intact and evenly distributed within the augmented tissue. In the upper third of the cylinders, the HA/TCP granules were mainly surrounded by non-mineralized tissue. In the two test groups, significantly more newly formed bone could be detected, partly reaching reached the upper third of the cylinder.

The area of bone regeneration on the sections of the cylinders was found to be as follows:

| | | Area of bone regeneration | |
|---|---|---|---|
| Condition | Number of samples | Mean (%) | SE |
| PEG-PTH 100 | 16 | 53.5 | 5.1 |
| PEG-PTH 20 | 14 | 51.1 | 5.4 |
| PEG | 16 | 34.3 | 5.1 |
| empty | 15 | 23.2 | 5.2 |

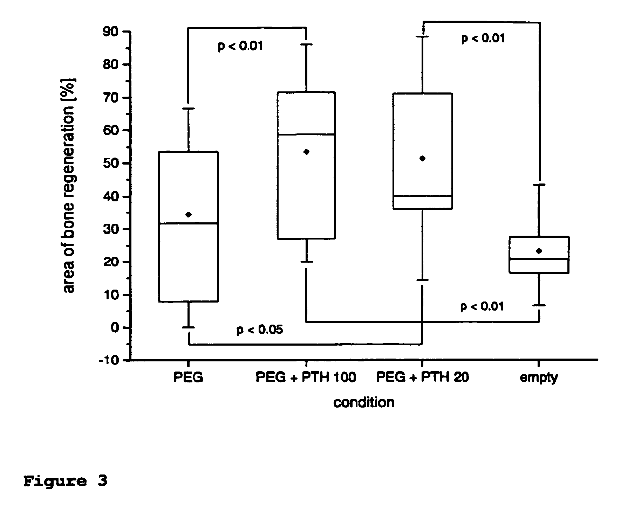

Figure 3 shows the areas of bone regeneration for the different treatments as well as the significance levels. From these data, it is concluded that the combination of a granulate and a polyethylene glycol hydrogel containing a covalently bound peptide of the parathyroid hormone combined with HA/TCP granules significantly stimulates in situ bone augmentation in rabbits.

Specifically, Figure 3 shows the area of bone regeneration for the rabbit cranial cylinder model applying granules with PEG. Values are displayed as box-plots ranging from the 25^{th} to the 75^{th} quantiles, including the median and whiskers extending 1.5 times the interquartile range.

### Example 5

### Methods

8 adult (12 months old) New Zealand White rabbits, weighing between 3 and 4 kg, were anesthetized and obtained each 4 titanium cylinders of 7 mm in height and 7 mm in outer diameter, which were screwed in 1 mm deep circular perforated slits made in the cortical bones of the calvaria. The following 4 treatment modalities were randomly allocated: (1) empty control, (2) a combination of PEG matrix containing 0.31 mg/ml covalently bound RGD and hydroxyapatite (HA) / tricalciumphosphate (TCP) granules (Straumann Bone Ceramic; Institut Straumann AG, Basel, Switzerland), and a combination of PEG matrix containing 0.31 mg/ml covalently bound RGD and either 15 µg (3) or 30 µg (4) of non-bound recombinant BMP-2 and HA/TCP granules.

Immediately before application, 4-arm PEG-acrylate 15k and HS-PEG-SH 3.4k (both from Nektar, Huntsville, AL, USA) were dissolved in 2 mM aqueous HCl solution to yield a homogeneous solution containing equimolar numbers of acrylate and thiol groups, which was then sterile filtered. Aliquots of the sterile PEG solution, a solution of a 9 amino acid cys-RGD peptide (Bachem, Bubendorf, Switzerland), and a BMP-2 solution were combined with a 0.4 M triethanolamine/HCl buffer (pH 8.85) to yield 204 µl of a solution containing 9.8 wt% PEG, 0.31 mg/ml RGD, and 0, 74, or 147 µg/ml BMP-2. This solution was then applied onto 150 mg of HA/TCP granules and mixed for about 10 seconds. Subsequently, this granular putty was applied into the determined cylinders. Within 60 seconds, the PEG gels set and thus stabilized the HA/TCP granules. The cylinders were left open towards the bone side but were closed with a titanium lid towards the covering skin-periosteal flap. The periosteum and the cutaneous flap were adapted and sutured for primary healing.

After 8 weeks, the animals were sacrificed and ground sections were prepared for histology.

The bone formation in the cylinders was evaluated histologically. Mean values and standard deviations were calculated for the amounts of bone formation within the cylinders, either evaluated by the point measurements or by the area of bone regeneration.

### Results

All animals showed uneventful healing of the area of surgery and no reductions in body weights were noted.

The area percentages of mineralized bone on the sections of the cylinders were found to be as follows:

| | | Mineralized bone | |
|---|---|---|---|
| Condition | Number of samples | Mean (%) | SD |
| PEG-BMP 30µg | 10 | 30.2 | 7.6 |
| PEG-BMP 15µg | 10 | 25.0 | 7.9 |
| PEG | 10 | 15.2 | 8.0 |
| empty | 9 | 13.9 | 5.7 |

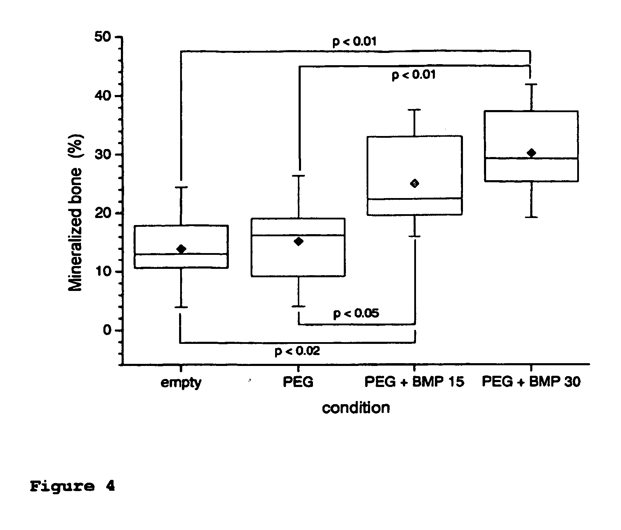

Figure 4 shows the area percentages of bone regeneration for the different treatments as well as the significance levels. From these data, it is concluded that the combination of a granulate and a polyethylene glycol hydrogel containing a covalently bound RGD peptide and entrapped BMP-2, combined with HA/TCP granules significantly stimulates in situ bone augmentation in rabbits.

Specifically figure 4 shows the percentages of mineralized bone found for the rabbit cranial cylinder model applying granules with PEG. Values are displayed as box-plots ranging from the 25^{th} to the 75^{th} quantiles, including the median and whiskers extending 1.5 times the interquartile range.

## Claims

1. A biocompatible composition comprising
30 to 60% by weight of a granulate of synthetic materials selected from the group of hydroxyapatite, tricalciumphosphate, mixtures of hydroxyapatite and tricalciumphosphate, bioactive glass, calcium sulfate and carbonate apatite and a matrix formed by a self selective reaction of at least two precursors A and B in the presence of water, wherein
a first precursor A comprises a core carrying n chains each having a terminal conjugated unsaturated group or a terminal conjugated unsaturated bond and
a second precursor B comprises a core carrying m chains each having a terminal thiol group, wherein
m is greater than or equal to 2,
n is greater than or equal to 2, and
m+n is greater than or equal to 5.

2. Composition according to claim 1, wherein the granulate comprises hydroxyapatite and tricalciumphosphate.

3. Composition according to claim 1, wherein the granulate comprises hydroxyapatite and/or tricalcium phosphate.

4. Composition according to claim 1 to 3, wherein the weight ratio of hydroxyapatite/tricalciumphosphate in the granulate is between 0.1 to 5.0, preferably between 1.0 to 4.0, most preferably between 1.0 to 2.0.

5. Composition according to claim 1 to 4, wherein the content of hydroxyapatite in the granulate is at least 1% by weight, preferably equal to or more than 15% by weight, most preferably equal to or more than 50% by weight.

6. Composition according to claim 1 to 5, wherein the core of precursor A is a carbon atom, a nitrogen atom, ethylene oxide, an amino acid or a peptide, a carbohydrate, a multifunctional alcohol, glycerol or oligoglycerol.

7. Composition according to claim 1 to 6, wherein the core of precursor B is a carbon atom, a nitrogen atom, ethylene oxide, an amino acid or a peptide, a carbohydrate, a multifunctional alcohol, glycerol or oligoglycerol.

8. Composition according to claim 6, wherein the core of precursor A is a carbon atom, an ethylene oxide unit, glucose, D-sorbitol, pentaerythritol, glycerol or hexaglycerol.

9. Composition according to claim 7, wherein the core of precursor B is a carbon atom, an ethylene oxide unit, a peptide, glucose, D-sorbitol, pentaerythritol, glycerol or hexaglycerol.

10. Composition according to claim 9, wherein the peptide comprises one or more enzymatic degradation sites.

11. Composition according to claim 1 to 10, wherein precursor B comprises a peptide which comprises one or more enzymatic degradation sites.

12. Composition according to claim 1 to 11, comprising a third precursor which comprises or is a peptide, wherein the peptide comprises one or more enzymatic degradation sites.

13. Composition according to claim 1 to 12, wherein the conjugated unsaturated group or the conjugated unsaturated bond of first precursor A is an acrylate, an acrylamide, a quinine, a 2- or 4-vinylpyridinium, vinylsulfone, maleimide or an itaconate ester.

14. Composition according to claim 13, wherein the conjugated unsaturated group or the conjugated unsaturated bond of first precursor A is an acrylate.

15. Composition according to claim 1 to 14, wherein the first precursor A is selected from the group consisting of

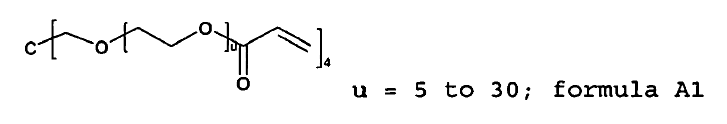

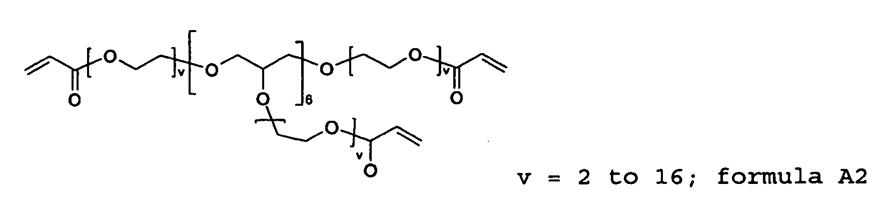

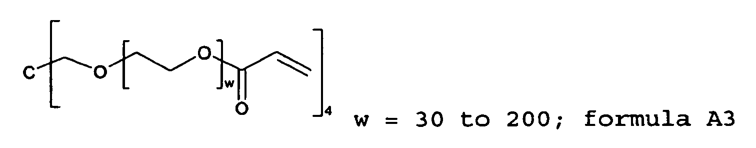

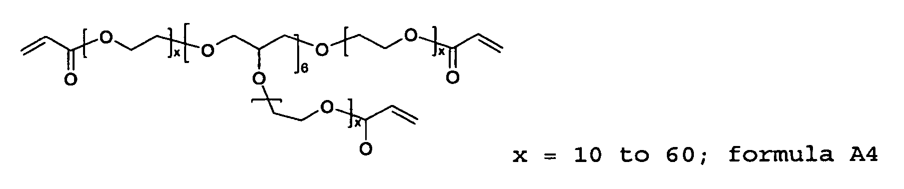

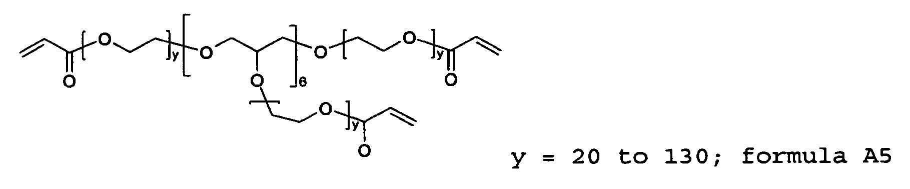

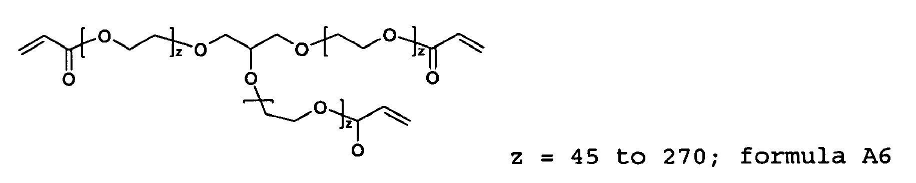

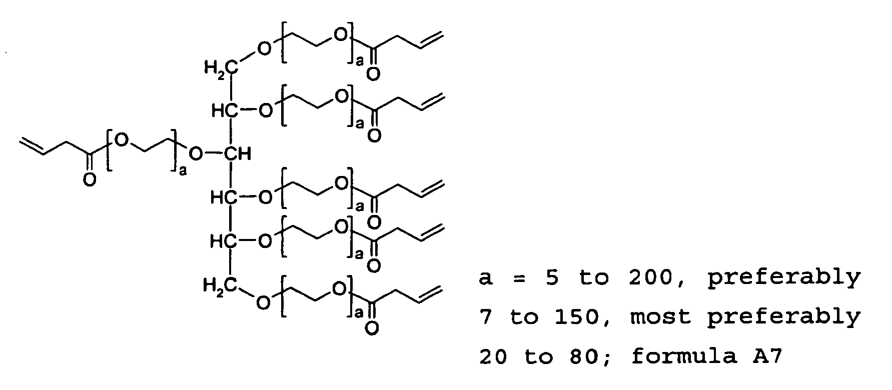

16. Composition according to claim 1 to 15, wherein the precursor B comprises a thiol moiety.

17. Composition according to claim 1 to 16, wherein the second precursor B is selected from the group consisting of

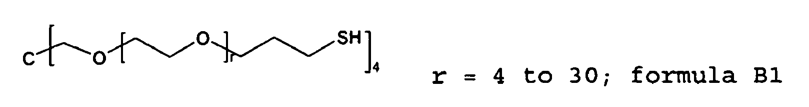

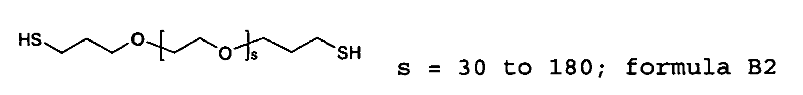

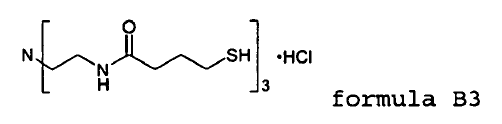

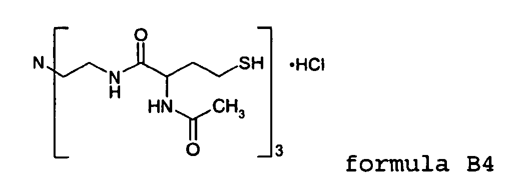

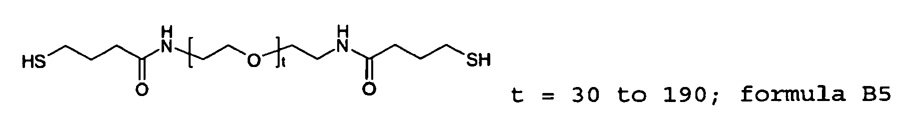

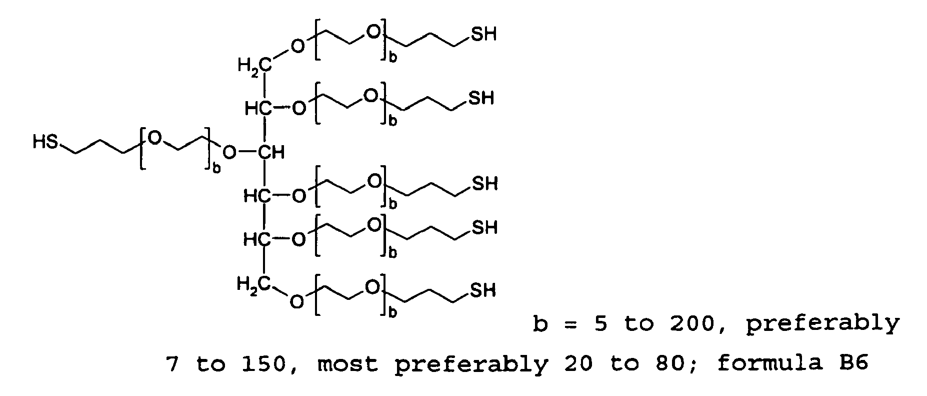

18. Composition according to claim 1 to 17, wherein the precursors A and/or B comprise chains having a molecular weight between 500 and 100'000 Da, preferably between 1000 and 50'000 Da, most preferably between 2000 and 30'000 Da.

19. Composition according to claim 1 to 18, wherein the chains of precursor A and/or B are a polymer selected from the group consisting of poly(vinyl alcohol), poly(alkylene oxides), poly(ethylene glycol), poly(oxyethylated polyols), poly(oxyethylated sorbitol), poly(oxyethylated glucose), poly(oxazoline), poly(acryloyl-morpholine), poly(vinylpyrrolidone), and mixtures thereof.

20. Composition according to claim 1 to 19, wherein the chains of precursor A and/or B are poly(ethylene glycol).

21. Composition according to claim 1 to 20, wherein the composition comprises at least one bioactive factor covalently bound to the matrix or entrapped in the composition.

22. Composition according to claim 21, wherein the bioactive factor comprises a thiol.

23. Composition according to claim 21 or 22, wherein the bioactive factor is selected from the group consisting of parathyroid hormones (PTH), peptides based on PTH, peptide fragments of PTH, peptides comprising a RGD tripeptide, transforming growth factor beta family (TGFβ), bone morphogenetic protein family (BMP), platelet derived growth factor family (PDGF), vascular endothelial growth factor family (VEGF), insulin like growth factor family (IGF), fibroblast growth factor family (FGF), enamel matrix derivative proteins and peptides (EMD), prostaglandin E₂ (PGE₂) and dentonin.

24. Composition according to claim 23, wherein the bioactive factor is selected from the group consisting of parathyroid hormones (PTH), peptides based on PTH and peptide fragments of PTH.

25. Composition according to claim 23 or 24, wherein the bioactive factor is Cys (PTH₁₋₃₄).

26. Composition according to claim 23, wherein the enamel matrix derivative protein or peptide (EMD) is selected from the group consisting of amelogenin, amelin, tuftelin, ameloblastin, enamelin and dentin sialoprotein.

27. Composition according to claim 23, wherein the bioactive factor is H-Gly-Cys-Gly-Arg-Gly-Asp-Ser-Pro-Gly-NH₂.

28. Kit for preparing a composition according to claim 1 to 27 comprising
(i) 30 to 60% by weight of a granulate, and
(ii) a precursor A, and
(iii) a precursor B,
wherein the granulate, precursor A and precursor B are as defined in claim 1, as individually stored components.

29. Kit according to claim 28 comprising
(iv) at least one bioactive factor, wherein the bioactive factor is defined according to claims 19 to 25, as a further individually stored component.

30. Kit according to claim 28 or 29, wherein the granulate and precursor A are stored as premix.

31. Kit according to claim 28 or 29, wherein the granulate and precursor B are stored as premix.

32. Kit according to claim 29, wherein the precursor B and the bioactive factor are stored as premix.

33. Kit according to claim 29, wherein the bioactive factor is stored in lyophilized form.

34. Kit according to claim 29, wherein the bioactive factor is stored in suitable buffer solution.

35. Method for preparing a composition according to claim 1 to 27 by mixing the granulate, the precursor A and the precursor B in the presence of water in a suitable container.

36. Method for preparing a composition according to claim 1 to 27 by
a) mixing the precursor A, the precursor B and the bioactive factor in the presence of water in a suitable container, and
b) adding and mixing the granulate with the premix obtained in step a).

37. Composition according to claim 1 to 27 for use as a bone repair and/or bone augmentation material.

## Patentansprüche

1. Eine biokompatible Zusammensetzung umfassend
30-60 Gew.% eines Granulats eines synthetischen Materials ausgewählt aus der Gruppe von Hydroxylapatit, Trikalziumphosphat, Mischungen von Hydroxylapatit und Trikalziumphosphat, bioaktivem Glas, Kalziumsulfat und Karbonatapatit und einer Matrix gebildet durch eine eigenselektive Reaktion von mindestens zwei Vorläufern A und B in der Gegenwart von Wasser, wobei
ein erster Vorläufer A einen Kern umfasst, welcher n Ketten trägt, wobei jede Kette eine terminale konjugierte ungesättigte Gruppe oder eine terminale konjugierte ungesättigte Bindung aufweist, und
ein zweiter Vorläufer B einen Kern umfasst, welcher m Ketten trägt, wobei jede Kette eine terminale Thiolgruppe aufweist, wobei
m grösser oder gleich 2 ist,
n grösser oder gleich 2 ist, und
m+n grösser oder gleich 5 ist.

2. Zusammensetzung gemäss Anspruch 1, wobei das Granulat Hydroxylapatit und Trikalziumphosphat umfasst.

3. Zusammensetzung gemäss Anspruch 1, wobei das Granulat Hydroxylapatit und/oder Trikalziumphosphat umfasst.

4. Zusammensetzung gemäss Anspruch 1 bis 3, wobei das Gewichtsverhältnis von Hydroxylapatit/Trikalziumphosphat im Granulat zwischen 0.1 bis 5.0 ist, bevorzugt zwischen 1.0 bis 4.0, am meisten bevorzugt zwischen 1.0 und 2.0.

5. Zusammensetzung gemäss Anspruch 1 bis 4, wobei der Gehalt an Hydroxylapatit im Granulat mindestens 1 Gew.% ist, bevorzugt gleich oder mehr als 15 Gew.%, am meisten bevorzugt gleich oder mehr als 50 Gew.%.

6. Zusammensetzung gemäss Anspruch 1 bis 5, wobei der Kern des Vorläufers A ein Kohlenstoffatom, ein Stickstoffatom, Ethylenoxid, eine Aminosäure oder ein Peptid, ein Kohlenhydrat, ein multifunktioneller Alkohol, Glycerol oder Oligoglycerol ist.

7. Zusammensetzung gemäss Anspruch 1 bis 6, wobei der Kern des Vorläufers B ein Kohlenstoffatom, ein Stickstoffatom, Ethylenoxid, eine Aminosäure oder ein Peptid, ein Kohlenwasserstoff, ein multifunktioneller Alkohol, Glycerol oder Oligoglycerol ist.

8. Zusammensetzung gemäss Anspruch 6, wobei der Kern des Vorläufers A ein Kohlenstoffatom, eine Ethylenoxid-Einheit, Glucose, D-Sorbitol, Pentaerythritol, Glycerol oder Hexaglycerol ist.

9. Zusammensetzung gemäss Anspruch 7, wobei der Kern des Vorläufers B ein Kohlenstoffatom, eine Ethylenoxid-Einheit, ein Peptid, Glucose, D-Sorbitol, Pentaerythritol, Glycerol oder Hexaglycerol ist.

10. Zusammensetzung gemäss Anspruch 9, wobei das Peptid eine oder mehrere enzymatische Abbauregionen umfasst.

11. Zusammensetzung gemäss Anspruch 1 bis 10, wobei der Vorläufer B ein Peptid umfasst, welches eine oder mehrere enzymatische Abbauregionen umfasst.

12. Zusammensetzung gemäss Anspruch 1 bis 11, umfassend einen dritten Vorläufer, der ein Peptid umfasst oder ist, wobei das Peptid eine oder mehrere enzymatische Abbauregionen umfasst.

13. Zusammensetzung gemäss Anspruch 1 bis 12, wobei die konjugierte ungesättigte Gruppe oder die konjugierte ungesättigte Bindung des ersten Vorläufers A ein Acrylat, ein Acrylamid, ein Quinin, ein 2- oder 4-Vinylpyridinium, Vinylsulfon, Maleimid oder ein Itaconatester ist.

14. Zusammensetzung gemäss Anspruch 13, wobei die konjugierte ungesättigte Gruppe oder die konjugierte ungesättigte Bindung des ersten Vorläufers A ein Acrylat ist.

15. Zusammensetzung gemäss Anspruch 1 bis 14, wobei der erste Vorläufer A ausgewählt ist aus der Gruppe bestehend aus

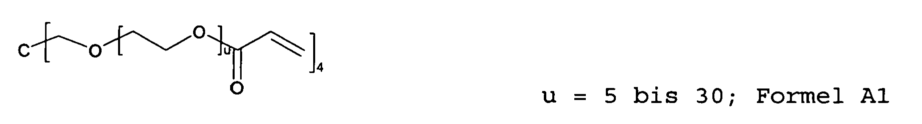

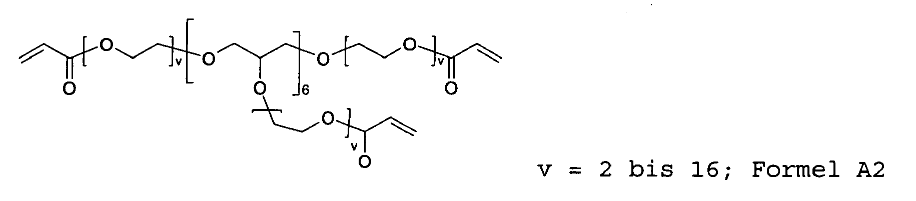

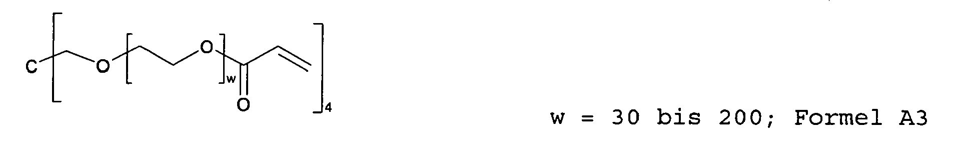

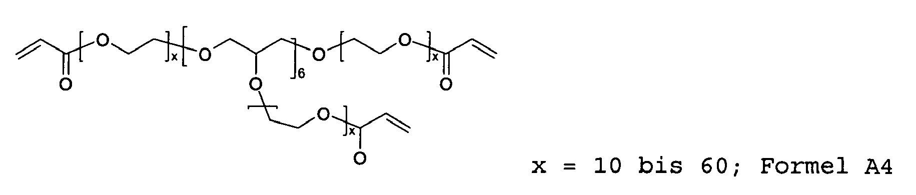

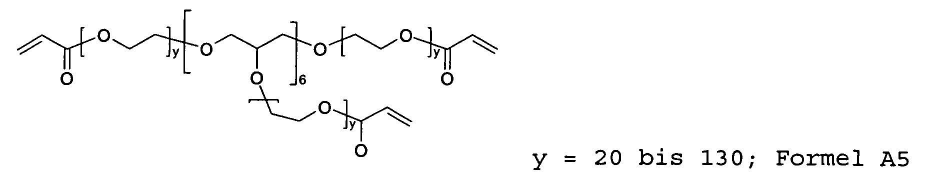

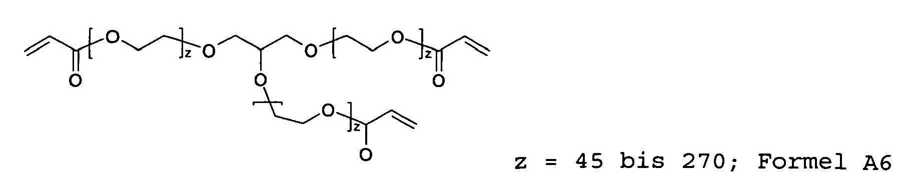

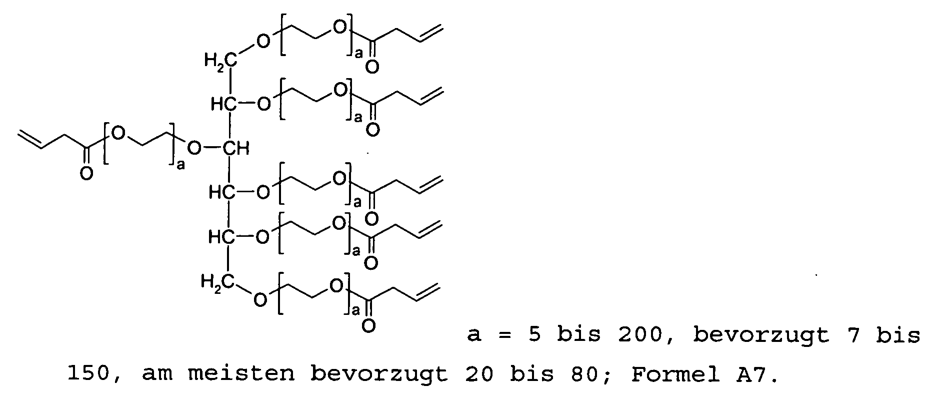

16. Zusammensetzung gemäss Anspruch 1 bis 15, wobei der Vorläufer B eine Thiolgruppe umfasst.

17. Zusammensetzung gemäss Anspruch 1 bis 16, wobei der zweite Vorläufer B ausgewählt ist aus der Gruppe bestehend aus

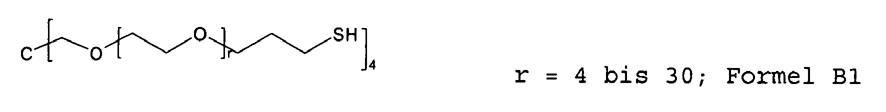

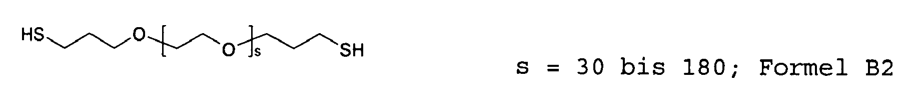

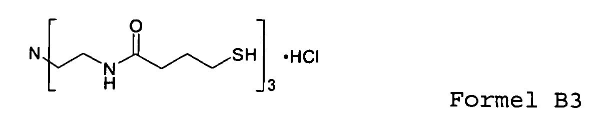

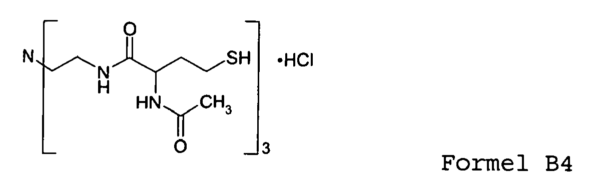

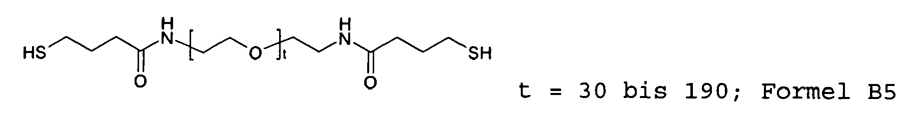

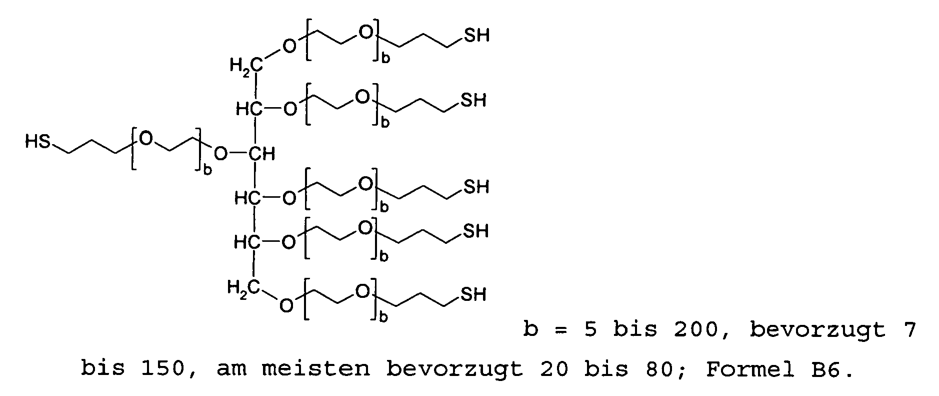

18. Zusammensetzung gemäss Anspruch 1 bis 17, wobei die Vorläufer A und/oder B Ketten umfassen, die ein Molekulargewicht von zwischen 500 und 100'000 Da haben, bevorzugt zwischen 1000 und 50'000 Da, am meisten bevorzugt zwischen 2000 und 30'000 Da.

19. Zusammensetzung gemäss Anspruch 1 bis 18, wobei die Ketten des Vorläufers A und/oder B ein Polymer sind, ausgewählt aus der Gruppe bestehend aus Poly-(Vinylalkohol), Poly-(Alkylenoxiden), Poly-(Ethylengycol), Poly-(oxyethylierten Polyolen), Poly-(oxyethyliertem Sorbitol), Poly-(oxyethylierter Glucose), Poly-(Oxazolin), Poly-(Acrylolylmorpholin), Poly-(Vinylpyrrolidon), und Mischungen davon.

20. Zusammensetzung gemäss Anspruch 1 bis 19, wobei die Ketten des Vorläufers A und/oder B Poly-(Ethylenglycol) sind.

21. Zusammensetzung gemäss Anspruch 1 bis 20, wobei die Zusammensetzung mindestens einen bioaktiven Faktor umfasst, der kovalent an die Matrix gebunden oder in der Zusammensetzung eingeschlossen ist.

22. Zusammensetzung gemäss Anspruch 21, wobei der bioaktive Faktor ein Thiol umfasst.

23. Zusammensetzung gemäss Anspruch 21 oder 22, wobei der bioaktive Faktor ausgewählt ist aus der Gruppe bestehend aus Parathyroidhormonen (PTH), Peptiden basierend auf PTH, Peptidfragmenten von PTH, Peptiden beinhaltend ein RGD Tripeptid, der transformierenden Wachstumsfaktorbeta-Familie (BGFβ), der knochenmorphogenetischen Proteinfamilie (BMP), der Blutplättchen-abgeleiteten Wachstumsfaktorfamilie (PDGF), der vaskulär-endothelialen Wachstumsfaktorfamilie (VEGF), der insulinähnlichen Wachstumsfaktorfamilie (IGF), der fibroblastischen Wachstumsfaktorfamilie (FGF), Enamel-Matrix-Derivat Proteinen und Peptiden (EMD), Prostaglandin E₂ (PGE₂) und Dentonin.

24. Zusammensetzung gemäss Anspruch 23, wobei der bioaktive Faktor ausgewählt ist aus der Gruppe bestehend aus Parathyroidhormonen (PTH), Peptiden basierend auf PTH und Peptidfragmenten von PTH.

25. Zusammensetzung gemäss Anspruch 23 oder 24, wobei der bioaktive Faktor Cys (PTH₁₋₃₄) ist.

26. Zusammensetzung gemäss Anspruch 23, wobei das Enamel-Matrix-Derivat Protein oder Peptid (EMD) ausgewählt ist aus der Gruppe bestehend aus Amelogenin, Amelin, Tuftelin, Ameloblastin, Enamelin und Dentin-Sialoprotein.

27. Zusammensetzung gemäss Anspruch 23, wobei der bioaktive Faktor H-Gly-Cys-Gly-Arg-Gly-Asp-Ser-Pro-Gly-NH₂ ist.

28. Kit zur Herstellung einer Zusammensetzung gemäss Anspruch 1 bis 27, umfassend
(i) 30 bis 60 Gew.% eines Granulats, und
(ii) ein Vorläufer A, und
(iii) ein Vorläufer B,
wobei das Granulat, der Vorläufer A und der Vorläufer B so sind wie in Anspruch 1 definiert, als einzeln aufbewahrte Bestandteile.

29. Kit gemäss Anspruch 28 umfassend
(iv) mindestens einen bioaktiven Faktor, wobei der bioaktive Faktor definiert ist gemäss Anspruch 19 bis 25, als weiteren einzeln aufbewahrten Bestandteil.

30. Kit gemäss Anspruch 28 oder 29, wobei das Granulat und der Vorläufer A als Vormischung aufbewahrt sind.

31. Kit gemäss Anspruch 29, wobei das Granulat und der Vorläufer B als Vormischung aufbewahrt sind.

32. Kit gemäss Anspruch 28 oder 29, wobei der Vorläufer B und der bioaktive Faktor als Vormischung aufbewahrt sind.

33. Kit gemäss Anspruch 29, wobei der bioaktive Faktor in lyophilisierter Form aufbewahrt ist.

34. Kit gemäss Anspruch 29, wobei der bioaktive Faktor in einer geeigneten Pufferlösung aufbewahrt ist.

35. Verfahren zur Herstellung einer Zusammensetzung gemäss Anspruch 1 bis 27 durch Mischen des Granulats, des Vorläufers A und des Vorläufers B in Gegenwart von Wasser in einem geeigneten Behälter.

36. Verfahren zur Herstellung einer Zusammensetzung gemäss Anspruch 1 bis 27 durch
a) Mischen des Vorläufers A, des Vorläufers B und des bioaktiven Faktors in Gegenwart von Wasser in einem geeigneten Behälter, und
b) Hinzufügen und Mischen des Granulats mit der aus Schritt a) erhaltenen Vormischung.

37. Zusammensetzung gemäss Anspruch 1 bis 27 zum Gebrauch als ein knochenreparierendes und/oder knochenaufbauendes Material.

## Revendications

1. Composition biocompatible comprenant de 30 à 60 % en poids d'un granulat de matériaux synthétiques choisis dans le groupe constitué de l'hydroxyapatite, du phosphate tricalcique, de mélanges d'hydroxyapatite et de phosphate tricalcique, de verre bioactif, de sulfate de calcium et de carbonate-apatite et une matrice formée par une réaction autosélective d'au moins deux précurseurs A et B en présence d'eau,
un premier précurseur A comprend un noyau comportant n chaînes ayant chacune un groupe insaturé conjugué terminal ou une liaison insaturée conjuguée terminale, et
un deuxième précurseur B comprend un noyau comportant m chaînes ayant chacune un groupe thiol terminal, où
m est supérieur ou égal à 2,
n est supérieur ou égal à 2, et
m+n est supérieur ou égal à 5.

2. Composition selon la revendication 1, dans laquelle le granulat comprend de l'hydroxyapatite et du phosphate tricalcique.

3. Composition selon la revendication 1, dans laquelle le granulat comprend de l'hydroxyapatite et/ou du phosphate tricalcique.

4. Composition selon les revendications 1 à 3, dans laquelle le rapport en poids d'hydroxyapatite / phosphate tricalcique dans le granulat est compris entre 0.1 à 5.0, de préférence entre 1.0 à 4.0, de manière préférée entre toutes entre 1.0 à 2.0.

5. Composition selon les revendications 1 à 4, dans laquelle la teneur en hydroxyapatite dans le granulat est d'au moins 1 % en poids, de préférence égale ou supérieure à 15 % en poids, de manière préférée entre toutes égale ou supérieure à 50 % en poids.

6. Composition selon les revendications 1 à 5, dans laquelle le noyau du précurseur A est un atome de carbone, un atome d'azote, l'oxyde d'éthylène, un acide aminé ou un peptide, un glucide, un alcool multifonctionnel, le glycérol ou un oligoglycérol.

7. Composition selon les revendications 1 à 6, dans laquelle le noyau du précurseur B est un atome de carbone, un atome d'azote, l'oxyde d'éthylène, un acide aminé ou un peptide, un glucide, un alcool multifonctionnel, un glycérol ou un oligoglycérol.

8. Composition selon la revendication 6, dans laquelle le noyau du précurseur A est un atome de carbone, un groupe oxyde d'éthylène, un glucose, un D-sorbitol, un pentaérythritol, un glycérol ou un hexaglycérol.

9. Composition selon la revendication 7, dans laquelle le noyau de précurseur B est un atome de carbone, un groupe oxyde d'éthylène, un peptide, un glucose, un D-sorbitol, un pentaérythritol, un glycérol ou un hexaglycérol.

10. Composition selon la revendication 9, dans laquelle le peptide comprend un ou plusieurs sites de dégradation enzymatique.

11. Composition selon les revendications 1 à 10, dans laquelle le précurseur B comprend un peptide qui comprend un ou plusieurs sites de dégradation enzymatique.

12. Composition selon les revendications 1 à 11, comprenant un troisième précurseur qui comprend ou est un peptide, où le peptide comprend un ou plusieurs sites de dégradation enzymatique.

13. Composition selon les revendications 1 à 12, dans laquelle le groupe insaturé conjugué ou la liaison insaturée conjuguée du premier précurseur A est un acrylate, un acrylamide, une quinine, un 2- ou 4-vinylpyridinium, une vinylsulfone, un maléimide ou un ester d'itaconate.

14. Composition selon la revendication 13, dans laquelle le groupe insaturé conjugué ou la liaison insaturée conjuguée du premier précurseur A est un acrylate.

15. Composition selon les revendications 1 à 14, dans laquelle le précurseur A est choisi dans le groupe constitué de

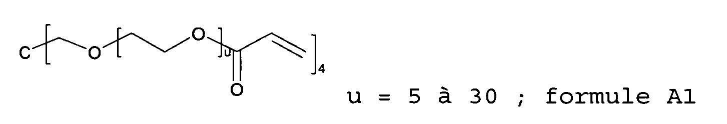

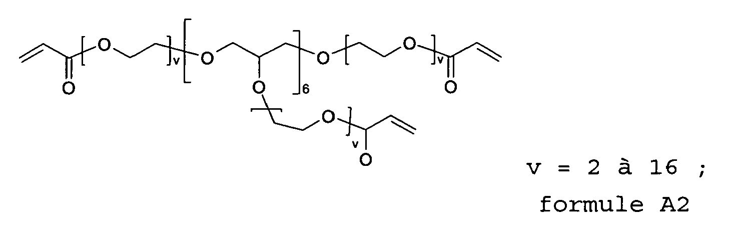

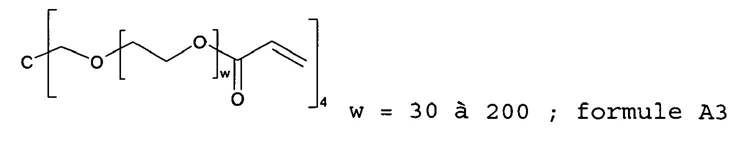

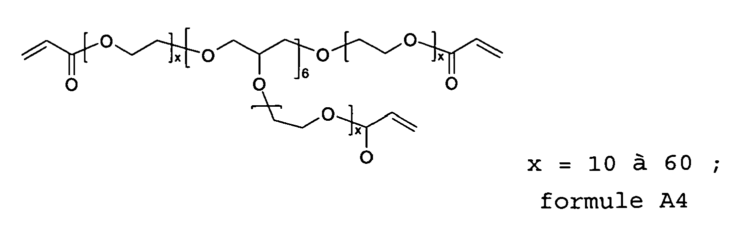

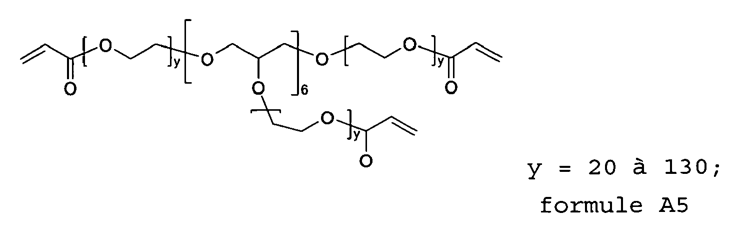

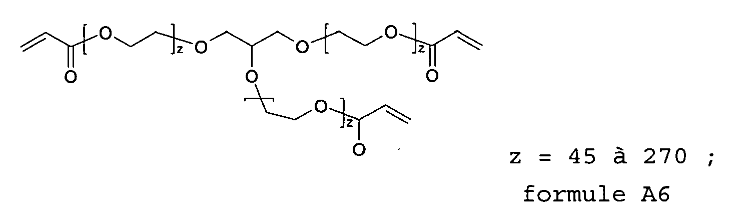

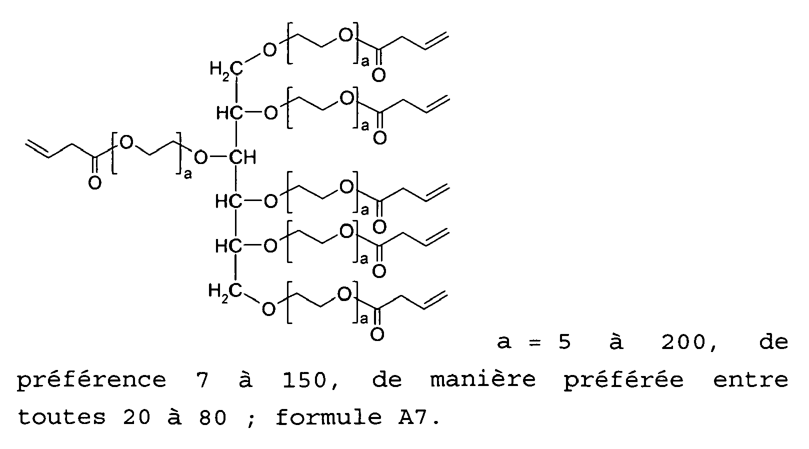

16. Composition selon les revendications 1 à 15, dans laquelle le précurseur B comprend un fragment thiol.

17. Composition selon les revendications 1 à 16, dans laquelle le deuxième précurseur B est choisi dans le groupe constitué de

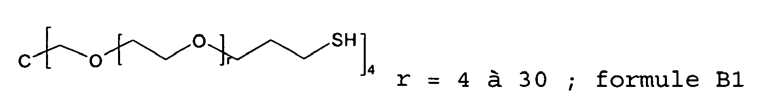

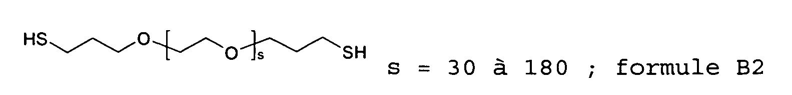

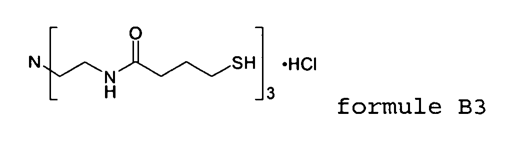

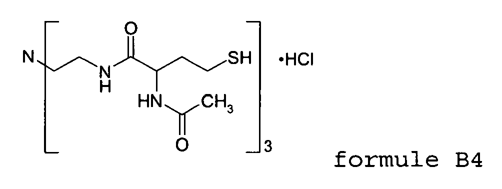

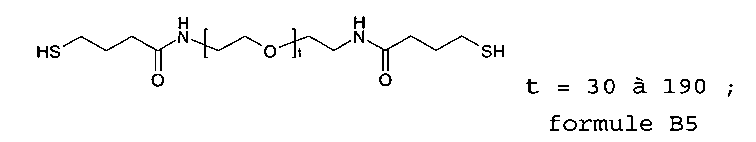

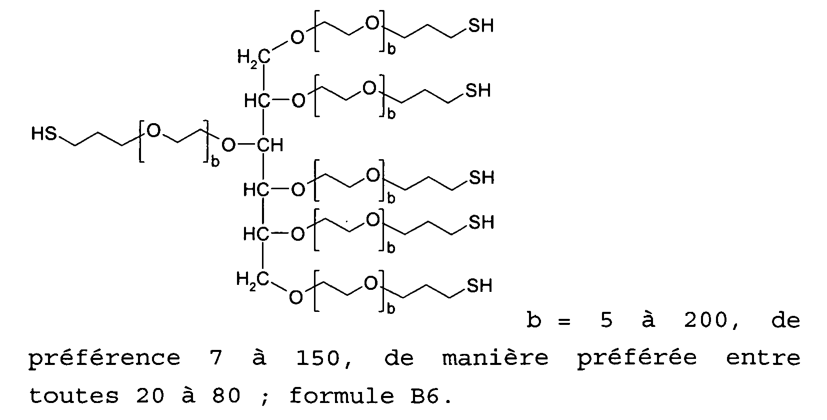

18. Composition selon les revendications 1 à 17, dans laquelle les précurseurs A et/ou B comprennent des chaînes ayant un poids moléculaire compris entre 500 et 100 000 Da, de préférence entre 1 000 et 50 000 Da, de manière préférée entre toutes entre 2000 et 30 000 Da.

19. Composition selon les revendications 1 à 18, dans laquelle les chaînes des précurseurs A et/ou B sont un polymère choisi dans le groupe constitué de poly(alcool vinylique), poly(oxydes d'alkylène), poly(éthylèneglycol), poly(polyols oxyéthylés), poly(sorbitol oxyéthylé), poly(glucose oxyéthylé), poly(oxazoline), poly(acryloyl-morpholine), poly(vinylpyrrolidone), et des mélanges de ceux-ci.

20. Composition selon les revendications 1 à 19, dans laquelle les chaînes des précurseurs A et/ou B sont du poly(éthylèneglycol).

21. Composition selon les revendications 1 à 20, dans laquelle la composition comprend au moins un facteur bioactif lié de façon covalente à la matrice ou piégé dans la composition.

22. Composition selon la revendication 21, dans laquelle le facteur bioactif comprend un thiol.

23. Composition selon la revendication 21 ou 22, dans laquelle le facteur bioactif est choisi dans le groupe constitué de parathormones (PTH), peptides à base de PTH, fragments peptidiques de PTH, peptides comprenant un tripeptide RGD, la famille de facteurs de croissance transformants bêta (TGFβ), la famille de protéines morphogénétiques osseuses (BMP), la famille de facteurs de croissance dérivés des plaquettes (PDGF), la famille de facteurs de croissance endothéliaux vasculaires (VEGF), la famille de facteurs de croissance insulinomimétiques (IGF), la famille de facteurs de croissance des fibroblastes (FGF), les protéines et peptides dérivés de la matrice d'émail (EMD), la prostaglandine E₂ (PGE₂) et la dentonine.

24. Composition selon la revendication 23, dans laquelle le facteur bioactif est choisi dans le groupe constitué de parathormones (PTH), peptides à base de PTH et fragments peptidiques de PTH.

25. Composition selon la revendication 23 ou 24, dans laquelle le facteur bioactif est Cys (PTH₁₋₃₄).

26. Composition selon la revendication 23, dans laquelle la protéine ou le peptide dérivé(e) de matrice d'émail (EMD) est choisi dans le groupe constitué de l'amélogénine, l'améline, la tuftéline, l'améloblastine, l'énaméline et la sialoprotéine dentine.

27. Composition selon la revendication 23, dans laquelle le facteur bioactif est H-Gly-Cys-Gly-*Arg*-*Gly-Asp*-Ser-Pro-Gly-NH₂.

28. Kit pour préparer une composition selon les revendications 1 à 27, comprenant
(i) de 30 à 60 % en poids d'un granulat, et
(ii) un précurseur A, et
(iii) un précurseur B,
où le granulat, le précurseur A et le précurseur B sont tels que définis dans la revendication 1, sous forme de composants conservés individuellement.

29. Kit selon la revendication 28, comprenant
(iv) au moins un facteur bioactif, où le facteur bioactif est défini selon les revendications 19 à 25, sous forme d'un composant supplémentaire conservé individuellement.

30. Kit selon la revendication 28 ou 29, dans lequel le granulat et le précurseur A sont conservés sous forme de prémélange.

31. Kit selon la revendication 28 ou 29, dans lequel le granulat et le précurseur B sont conservés sous forme de prémélange.

32. Kit selon la revendication 29, dans lequel le précurseur B et le facteur bioactif sont conservés sous forme de prémélange.

33. Kit selon la revendication 29, dans lequel le facteur bioactif est conservé sous forme lyophilisée.

34. Kit selon la revendication 29, dans lequel le facteur bioactif est conservé dans une solution tampon adaptée.

35. Procédé pour préparer une composition selon les revendications 1 à 27 par mélange du granulat, du précurseur A et du précurseur B en présence d'eau dans un récipient adapté.

36. Procédé pour préparer une composition selon les revendications 1 à 27 par
a) mélange du précurseur A, du précurseur B et du facteur bioactif en présence d'eau dans un récipient adapté, et
b) ajout et mélange du granulat avec le prémélange obtenu dans l'étape a).

37. Composition selon les revendications 1 à 27 pour utilisation en tant que matériau de réparation osseuse et/ou d'augmentation osseuse.
